# EUROPEAN PATENT SPECIFICATION

(11) **EP 3 769 822 B1**
(45) Date of publication and mention of the grant of the patent: **09.08.2023**
(21) Application number: 20173962.0
(22) Date of filing: 11.05.2020
(51) Int. Cl.: A63B 23/08, A61F 5/00, A61H 1/02, A61N 1/36, A63B 21/068, A63B 21/16

(54) **ANTISPASTIC DEVICE FOR EQUINOVARUS FOOT DEFORMITY**
ANTISPASTISCHE VORRICHTUNG FÜR KLUMPFUSS
APPAREIL ANTISPASTIQUE POUR PIED-BOT VARUS ÉQUIN

(30) Priority: 23.07.2019 UY 38313
(43) Date of publication of application: 27.01.2021
(73) Proprietor: Buccino Larronde, Luis Fernando, 3423 Montevideo (UY)
(72) Inventor: Buccino Larronde, Luis Fernando, 3423 Montevideo (UY)
(74) Representative: Balder IP Law, S.L.

(56) References cited:
- WO-A1-2018/190763
- DE-U1-202015 001 020
- FR-A1- 2 782 648
- JP-A- H 105 257
- KR-A- 20040 088 329

## Description

### FIELD OF THE INVENTION

The present invention refers to the field of physical rehabilitation, more specifically to the rehabilitation of neurological patients with spasticity.

### BACKGROUND

Spasticity has a neurological origin, generally due to CVA (Cerebrovascular Accident), MS (Multiple Sclerosis), SCI (Spinal Cord Injury), TBI (Traumatic Brain Injury), CP (Cerebral Palsy), genetic pathologies, etc.

Physiologically, spasticity can be defined as an increase in muscle tone, where the muscles are permanently contracted in an involuntary manner, causing resistance in passive movements and active movements (volitional movements). Spasticity causes deformities and the inability to perform basic motor tasks or the difficulty of achieving movements, for example walking; it causes losing balance in the standing position, and it is also possible that the spastic patient may suffer spasms (involuntary strong movements), causing pain and muscle-tendon shortening. Lower limbs with spasticity, particularly the foot, frequently adopt a deformed position called "spastic equinovarus foot", such deformity existing in three planes: frontal, sagittal and transverse.

The antispastic device of the present invention follows the law of the 1932 Nobel Prize-winning neurophysiologist, Charles Sherrington, in order to achieve the inhibition of spasticity, and allows us to learn about the physiology of muscle and the pathophysiology of spastic muscle. Inhibiting spasticity using passive stretching of the spastic musculature consists of moving away the points of origin and insertion (analytically) and the spastic muscular chain that includes the fasciae (systemically), thus, the inhibition of spasticity through stretching, which involves slow progressive movements, and maintained over time, targeting the three-dimensional area, that is, the three planes.

The existing devices for the treatment of spasticity currently known are tilted boards (conventional boards or elongation wedges, that is, "wedge board") used in the physical rehabilitation of neurological patients with foot spasticity. However wedge boards lack the ability of targeting the problem in a comprehensive manner. Thus, devices in the state of the art use wedge boards, with only one plane (sagittal plane, with Y axis of the Cartesian coordinates), without considering the verticality of the lower limb to be treated (LLTBT). Single-plane wedge boards do not solve the problem since the condition involves three planes.

Likewise, wedge boards do not have any additional technological system. In contrast to what is already known, the antispastic device of the present invention comprises kinematic measurements with movement sensors and the application of force sensors, for example electronic baropodometry. The existing wedge boards do not use laser light either for procuring the verticality of the LLTBT, which is corroborated from the sagittal and frontal planes, therefore no associated system to corroborate the verticality is included on wedge boards; additionally wedge boards do not corroborate the foot support in order to reproduce the angle of the wedge board. The antispastic device of the present invention, unlike the wedge board, provides data such as the surface of the plantigrade support or partial foot support (cm²), and it also recognizes where the maximum and average contact pressures are (g/cm²), for example if they are in the forefoot, midfoot, hindfoot, or if the foot is in pronation or supination or lateral or medial. The device also reports the foot load in Kg and the load percentage in the anatomical areas (movements on the same footprint), the load percentage in relation to the forefoot and hindfoot (talus and equinus support), the medial and lateral relationship load (pronation and supination), the medial-lateral arches, and the rotations of the foot in a transverse plane (adduction and abduction). In addition, information is obtained from the vectors with their own migration speed, direction, and sense of the maximum and average contact pressures during the rehabilitation session.

In the state of the art there are also arthromotor devices designed for passive movements. For example, the ankle arthromotor device is designed with 2 planes, frontal and sagittal. This device starts from the neutral position by making the movements in a single plane (for example, sagittal), ending the motion and moving back to the neutral position, then its starts again by making the movements in a single plane (for example, frontal); therefore it is not designed for both planes at the same time, nor does it use the transverse plane, that is, it does not move in a three-dimensional way, so this treatment does not apply to the fasciae, nor does it treat the soleus muscle or the posterior tibial muscle. Arthromotor devices do not receive body weight, unlike the device of the present invention, which is based on body weight and with the knee extended, this being a very important biomechanical factor to cover all the spastic muscles of the foot.

The biggest deficiency of arthromotor devices is the application of systemic movements that do not generate the sustained stretching of any muscle, so they do not cover the muscles that cause equinovarus foot and they do not access the foot fasciae either. The arthromotor device works with repetitions or cyclical movements. Some examples of arthromotor devices use electrostimulation of muscles that affect the movement of the arthromotor device, aiming at the activation of the muscles, without considering applying electrostimulation if the muscles are spastic, when it should inhibit the spasticity of the muscles applying currents in a reciprocal inhibition procedure using the antagonistic muscles of spasticity.

The ankle arthromotor device does not have a stretching position and it is used in the decubitus position, therefore, it does not receive the body weight; the pressure platforms cannot be used either and therefore it is not known whether spasticity is being inhibited. Thus, ankle arthromotor devices do not aim at inhibiting spasticity since none of these instruments moves in a three-dimensional form with full contact, and there is no ankle arthromotor device that is used with the knees extended and receiving the body weight. On the contrary, the antispastic device of the present invention includes the application of electrostimulation on the spasticity antagonist muscles, to generate reciprocal inhibition, while the arthromotor device does not use the spasticity antagonist muscles and does not remain in an inhibition position either. The arthromotor device does not allow joint ranges to be corrected with precision, because it does not allow sustained stretching in a three-dimensional form and it does not show stretching in one plane, two planes, nor three-dimensionally.

In the state of the art there is Document US4603687, which describes a device that is used passively with patient and it involves repetitive and predetermined basic movements; the ankle makes the dorsal flexion (one plane) and it is applied with a flexed knee.

Also, Document CN203954538 discloses a device designed to tone the foot and leg muscles of injured athletes, working from the ankle.

Document CN 204446517 discloses an instrument that uses fixed and variable pulleys to flex the knee with passive, basic, repetitive and predetermined movements, and it aims to flex the knee, seeking to improve spasms.

On the other hand, Document CN203724280 describes a pedal that performs the dorsal flexion (only in the sagittal plane) and which is applied with a flexed knee.

Document US2013245511 discloses a robotic device designed to do physical therapy exercises for patients with sequelae of stroke or other diseases, where the device involves simulation of a normal gait; during its use said device is based on repetitive movements, with a closed-loop and elliptical system and said regulation is described as an effect of spasticity reduction.

In turn, Document CN202236150 describes a bed or stretcher that moves parallel to the horizontal and moves towards the vertical, these movements being basic, passive, repetitive and predetermined. Such device is designed for ankle correction, it deals with only one plane (sagittal), and it has joint ranges in the sagittal plane from the neutral position to the triple-flexion position simultaneously (ankle-knee-hip joint).

Document FR2782648 relates to posture device for ankle and foot for bending, internal and external rotation, torsion and pulling of leg and foot muscles, comprising a horizontal support table where said table has the shape of a rectangular prism and contains attached a posterior supports insert structure generating different angles.

Document KR20040088329 discloses a stretching device for legs with a hinge pivot.

Thus, the present invention arises from the need to obtain a physical rehabilitation instrument that is capable of inhibiting spasticity in the foot, caused by neurological conditions, and that solves the deficiencies of the devices currently available in the market.

To this end, the device of the present invention involves a comprehensive approach for the inhibition of foot spasticity, with progressive and continuous three-dimensional stretches. The device allows placing the spastic foot on a pressure platform (baropodometry) that moves in all three planes, creating angles resulting in the joint ranges in the foot. The device of the present invention is used analytically and systemically and is designed beyond the spastic equinovarus foot, since it encompasses all spastic positions of the foot, for example: equinus, talus, pronation, supination, adduction and abduction. Thus, the device assesses, concludes and includes a rehabilitation process according to the patient and his evolution. It was designed after working on the rehabilitation of hundreds of patients and looking for a tool that greatly contributes to the rehabilitation procedures of those patients with neurological sequelae, whereby spastic equinovarus foot is quite often.

### BRIEF DESCRIPTION OF THE INVENTION

For the purposes of a better understanding of the invention, a series of terms are defined below that will be used throughout this specification:
Foot Abduction: moving away from the body midline in the transverse plane
Foot Adduction: approaching the body midline in the transverse plane
Descending/in descent: the forefoot is lower than the hindfoot
Ascending/in ascent: the forefoot is higher than the hindfoot
Equinus: plantar flexion, the foot is positioned downwards in the sagittal plane
Heterolateral: the right foot uses the sector of the device on the left and vice versa
Homolateral: the right foot uses the sector of the device on the right and vice versa.
RHD: Right Half Device
LHD: Left Half Device
LLTBT: Lower Limb to be treated
RF: Right foot
LF: Left foot.
Neutral position: articulation with 0° in all planes.
Joint ranges: degrees in which a joint moves in a plane.
Reduction: accessible joint ranges; maximum of a joint in a plane with a strong but slow and continuous movement, with the reduction procedure has a time of 10 seconds.
Assessment: assessment of the patient.
Valgus or pronation: support with the internal edge, or lateral edge in the frontal plane. Varus or supination: support with the external edge in the frontal plane.

The main objective of the device is to provide comprehensive rehabilitation and assessment procedures for the spastic equinovarus foot, so that by using it is possible to:
- Learn about the initial situation of the patient and monitor the rehabilitation process.
- Improve balance in standing position and when walking.
- Qualitatively inhibit spasticity by intensively reducing spastic hypertonia.
- Inhibit spasticity in a quantitative manner, targeting the muscle chain that causes the equinovarus foot, fasciae, and tendons.
- Achieve the sustainability of spastic reduction.
- Enhance the effects with the sustainability of botulinum toxin, drugs, conventional kinetic techniques, etc.
- Eliminate spasms in flexion and/or extension; correct deformities.
- Eliminate spontaneous Clonus and spontaneous Babinski sign; also achieve inhibition of flexing fingers (claw fingers from the second to the fifth).
- Fulfill a prophylactic function, protecting and preserving the spastic muscles, preserving muscle plasticity, avoiding muscle shortening, muscle fibrosis and joint restriction of the irreducible stage (joint ossification), as a result of rheological changes.
- Improve the strength of muscles that are antagonistic to spasticity.
- Correct the posture towards neutral position and also provide arthrokinetic information to correct body frame and gait pattern, assisting in the generation of a cortical motor engram.

The antispastic device object of the present invention is set out in the appended set of claims.

The antispastic device of the present invention provides information before starting rehabilitation. During the procedure (rehabilitation session) it provides the parameters that must be adjusted, and after the intervention (rehabilitation process). This information arises from an electronic baropodometry platform, which allows learning about the patient's condition and evolution during the rehabilitation process.

While in conventional rehabilitation treatments with stretching wedge boards for triceps surae muscle, a single plane wedge board is used, which accesses a single joint and does not cover all the muscles causing the equinovarus foot condition, the single plane wedge board of the present invention is manufactured to include all possible positions in each degree, for example an inclined plane (sagittal plane, Y axis of Cartesian coordinates) from 1° to 45° with 45 stretching positions.

Thus, the three-dimensional antispastic device of the present invention allows up to 16,200 positions to be generated, degree by degree in each of the possible positions, which results in 32,400 joint ranges in the tibiofibular-talar joint and talocalcaneal joint (45° multiplied by 360°) for the inhibition of spasticity of the spastic foot in a three-dimensional and comprehensive manner. In addition, it allows knowing with scientific rigor the evolution degree by degree, enabling the person in charge of the rehabilitation process to choose the necessary positions according to the patient's progress.

The muscles treated with conventional wedge boards are the calf and soleus muscles. These muscles achieve plantar flexion, which occurs in the tibiofibular-talar joint. The conventional wedge board does not cover the subtalar or talocalcaneal joint that occur in the frontal plane, nor does it cover the midtarsal joint that do not occur in the transverse plane, therefore, the tibialis posterior muscle cannot be accessed. The tibialis posterior muscle contracts to produce inversion, which takes place through supination, adduction, and it assists in plantar flexion, therefore it is the most important muscle for varus and it assists in the equinus position. Considering that spasticity appears as a spastic muscle chain, that spastic chain generates a postural conduct throughout all three planes, and therefore it is evident that the most comprehensive and simultaneous treatment for each plane, that is, a three-dimensional rehabilitation procedure, can only be achieved with the antispastic device of the present invention.

### Method of use

The initial assessment of the patient is carried out in two positions, in the decubitus position and in the standing (upright) position on the baropodometry platform of the antispastic device.

The decubitus position assessment studies joint ranges. It consists of passive and slow movements and is performed on all planes. Thus, the assessment procedure consists of an Accessible Joint Range Reduction (AJRR) and is performed in 10 seconds.

The selection and ranking of the patient is made by assessing the severity of the state of the muscles measured on the Ashworth scale, the level of spasticity of each muscle and the parameters of the baropodometry.

The reduction of ranges is a procedure carried out by the person in charge of the rehabilitation, since by knowing the restrictions and the possibilities of performing passive movements, the procedure for reducing joint ranges is captured through the 3D accelerometer electronic mobility sensor (which covers the 3 planes). Thus, the 3D sensor placed on the lower limb to be treated informs the passive movements accurately; in turn that data is uploaded into the software, which allows learning about the patient's clinical situation and progress after each rehabilitation procedure, and allows reproducing a 3D the foot image in the monitor.

For the proper use of the device of the present invention and achieving the aforementioned objectives, the patient must place the foot on a platform that corrects the spastic equinovarus foot through the specific three-dimensional stretching of certain muscles.

The device generates angles in space, and these angles result in joint ranges, so as to access all the patient's positions in space.

The procedure for using the antispastic device involves placing a foot with spastic equinovarus condition that receives the body weight on the platform. The lower limb to be treated must be in a knee extended and vertical position; while the other foot (which will not be treated) shall be minimally supported in a semi-flex position, placed on a board, bench, table or similar. Simultaneously, the patient has a fixed point to stabilize the upper limbs, for example, a Swedish ladder, so that the patient is comfortable, contributing to his overall balance and helping in putting the body weight on the lower limb to be treated.

The patient shall remain on the device for 3 series, according to the minutes and series provided in the kinetic program. For example, in the rehabilitation session the patient shall have a first series of 5 minutes with a 2 minute pause, a second series of 7 minutes followed with a 2 minute pause and finally, a third series of 10 minutes. Thus, each rehabilitation session involves at least 20 minutes using the device, without considering breaks. The increase in rehabilitation minutes is gradual, with 2 minute pauses every time.

If the patient fails to perform the rehabilitation procedure, that is, fails to remain in a vertical position for the time necessary to achieve reduction of spasticity, due to various factors, such as strength deficit or balance disorder, the antispastic device can be used with a stander, which prevents the device from being in a horizontal position, on the floor. The stander is associated with the device, that is, it moves together with it, copying the movements of the stander in the sagittal plane. Thus, the patient is positioned vertically in order to use 100% of the body weight.

Likewise it is possible to adjust the forces to be applied to the device, since they affect the tilted plane, creating 90 different forces (from horizontal to vertical), and in turn these forces affect each position of the device, allowing versatility in the rehabilitation procedure since it is possible to access to 16,200 different positions on the device platform, applying 90 forces in each position, reaching 1,458,000 possible situations in the rehabilitation procedure.

The patient is stabilized with straps from the trunk, pelvic girdle and extended knees and the stander has no footboard on the side where the antispastic device is used, and the limb that is not treated rests in a semi-flexed position on the table. Said stander moves from left to right and vice versa, and also the antispastic device moves from left and right and vice versa on the stander.

Likewise, with the stander at 90° on the sagittal plane, the percentage of body weight that the patient receives on the foot to be treated can be adjusted. In other words, using the stander, the patient can receive a percentage representing part of his body weight on the foot to be treated, adjusted according to the inclination, or 100% of his body weight.

### Device Utility

The antispastic device of the present invention is useful beyond neurological conditions, such as correction of the pronated or supinated foot and the overload on the equine forefoot or the varus hindfoot. This device allows modifying and balancing the pressure centers, the surface, the speed, etc.

The antispastic device also has other functions, such as relaxing muscles that are shortened, strained and in a situation of contracture; loss of balance leads to distortions in the distribution of the pressures center, the distribution of the load, and the usefulness of the parameters of the antispastic device to balance the agonist and antagonist muscle groups. Stretching achieves the relaxation of the shortened and strained muscles. In turn, weak muscle groups are toned with different forms of stimulation such as Russian electrical currents, Australian currents, diadynamic therapy, TENS, excitomotors, FES, EMS currents, in order to achieve reciprocal inhibition of the hypertonic muscle groups and an autogenous inhibition so as to balance towards normality. In other words, these transcutaneous currents allow meeting the objective of improving unbalanced muscle groups through vigorous contractions in weak muscle groups, and in turn allow the inhibition of the tone of muscle groups that are contracted and excessively strained.

The parameters obtained from the baropodometry platform are sent to the software to visualize the foot image on a 3D monitor at the same time as the foot contacts the platform. This allows observing the location of the pressure centers and the load on the foot sole, the magnitude of the inhibition and therefore learning when the inhibition or relaxation occurs (inhibition point). The 3D monitor shows the images of the muscles, with colors that differ between the muscles, shortened, flaccid, spastic, etc.

The antispastic device includes a series of electrostimulation inputs and waves which, upon their application on the agonist and antagonist spasticity muscle groups along with the stretching procedure that includes the fasciae, they fulfill two key objectives: inhibiting spastic groups and, in turn, improving the strength deficit of muscle groups antagonistic to spasticity. Thus, for example, the application of said inputs in the motor points of antagonistic muscles for reciprocal inhibition and the application of vibration on the tendons of the agonist (spastic) muscles enhance rehabilitation, obtaining a reduction in spasticity and the sustainability in the inhibition.

The aforementioned inputs can be excitomotor currents, Russian currents, Australian currents, transcutaneous and neuromuscular currents, and autogenous inhibition using vibrators to activate the Golgi tendon organ. These inputs are applied using an arm of the robotics attached to the antispastic device. Another technique for spasticity inhibition during stretching that can be used is cryotherapy.

Although some of these inputs were designed with another objective, it has been proven that they manage to assist the inhibition from the spastic agonist muscle itself when it is stretched, having significant results on the inhibition.

### BRIEF DESCRIPTION OF THE FIGURES

**FIGURE 1****:** shows the exploded view of the antispastic device for equinovarus foot deformity.
**FIGURE 2****:** shows the arrangement of the pieces for assembling the alpha (α) angle of the antispastic device for equinovarus foot deformity.
**FIGURE 3****:** shows the exploded view of the antispastic device to generate the beta (β) angle system on the beta angle generating support **T2,** in its settings for the treatment of the right foot and the left foot, respectively.

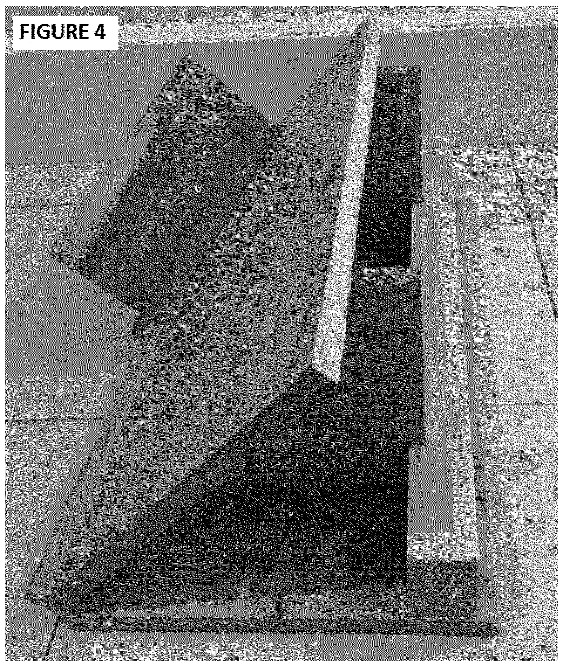

**FIGURE 4****:** shows a side view of the device assembled and ready to be used.
**FIGURE 5****:** shows a diagram with the beta angles (central axis) on the platform, with as much space as possible for the right foot.
**FIGURE 6****:** shows a diagram with the beta angles (central axis) on the platform, with as much space as possible for the left foot.

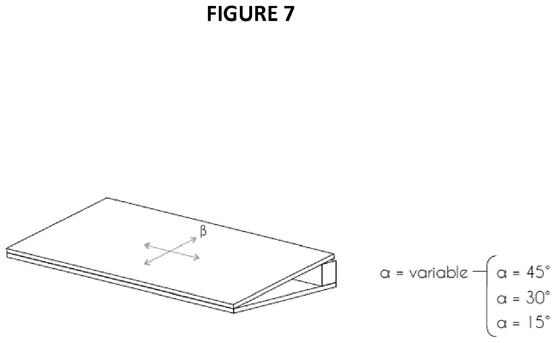

**FIGURE 7****:** shows a model of the antispastic device with its central axis on the beta angle support platform (360°).

### DETAILED DESCRIPTION OF THE INVENTION

Unlike already known devices, the antispastic device of this invention is thought and designed to act in a three-dimensional way, and is operated manually and/or mechanically, by means of electric motors or pneumatic pistons.

The antispastic device further comprises a PC tower and a 3D monitor, where it receives data from the sensors of the baropodometry platform and the 3D accelerometer electronic three-dimensional mobility sensor. These inputs are directly connected to the software to process the data obtained.

The monitor uses all three dimensions, that is, it places the foot in space, and it includes the three-dimensional vision in each joint (tibiofibular-talar, talocalcaneal and midtarsal joints) in each plane, and thus each person in charge of rehabilitation is able to make decisions or follow the program from the software. 3D images may be rotated 360°, for example, allowing a sagittal cut, or in every degree of the 360° to gain a better understanding of the muscles. The 3D monitor also shows the ranges of each joint and the electronic goniometer is observed on the screen on the chosen cut.

The position of the ranges in space is translated simply by following the points of origin and points of insertion, as well as the zero ranges or neutral or normal position. For example, the foot starts in the zero range and performs a movement in the sagittal plane of 30° dorsiflexing with the plantigrade footprint, the software captures and processes it to display the muscles on the 3D monitor.

By using the antispastic device of the present invention, it is possible to treat a selected and hierarchized muscle, being able to intervene prioritizing one muscle over another, with precise and three-dimensional elongations, in order to comprehensively treat the spastic foot condition.

The software detects the major inhibition points and reports all significant changes, as well as describes the parameters of each input simultaneously with the elongations, including shock waves, ultrasound, current type, thermotherapy, cryotherapy, and joint ranges degrees with the electronic goniometer.

For example, parameters such as patient exposure time, type of current to generate reciprocal inhibition, current intensity, focused, radial, and de-focused shock waves, and parameters of frequency (Hz), energy (mJ/mm²), impulses (number of impulses), Hz frequency of the vibrator for autogenous inhibition, come together to act advancing with the most demanding elongations from the antispastic device (always considering the physiological joint ranges and based on the AJRR of the initial assessment).

The device may also use the Cartesian coordinate system to move the platform mechanically using zippers, handles or electric motors. Likewise, the platform may be moved three-dimensionally by means of pistons from below or from above to achieve movement in all three planes (sagittal, frontal, transverse), which allows covering the necessary space, preserving the verticality of the leg to be treated and with the knee in extension.

The device of the present invention further comprises a series of inputs that, applied simultaneously to the antispastic device, comprehensively target spasticity:
1) Australian, Russian, FES, TENS, excitomotor, diadynamic currents: which are applied to the antagonistic muscle groups of spasticity, causing reciprocal inhibition; likewise muscles that receive the currents help to promote volitional activity and the gain strength. All currents that achieve vigorous contraction lead to reduced spasticity through reciprocal inhibition (by reciprocal innervation).

Artificial contraction produces tension in all directions, in the entire muscle and connective tissue. This effect reaches the deepest layers of muscle tissue, modifying the spastic pattern of the muscles. The use of simultaneous currents assisting elongation provides a significant plus for the reduction of spasticity. The contraction of the muscles with rehabilitation currents can be much stronger than the strength produced voluntarily.

From the pathophysiological point of view, spasticity has three main elements:
- Hyperexcitability of alpha and gamma motor neurons.
- Loss of presynaptic inhibition on the fibers (la).
- Interneurons manifest in distortion, there is a loss of rearrangement of neuronal activity in the medullary circuit; abnormal motor disorder occurs when there is spasticity, losing control of nerve impulses, both of inhibition and of excitatory potentials, receiving erroneous peripheral and supra-medullary impulses.

The vigorous currents applied on the spasticity antagonist muscles, correct the distortion in the medullary interneurons, since the procedure uses the interneurons directly. Said interneuronal correction occurs after rehabilitation with simultaneous inputs, for example with currents applying temporal and spatial temporal summation both to inhibit or excite, showing significant benefits, which can be interpreted as an adaptive process in the interneurons, so, when reciprocal inhibition is applied, there is an inhibition encompassing the alpha and gamma neuron-motor coactivation of the spastic muscle.

These inhibition techniques are based on the knowledge of Bernard Katz, Nobel Prize neurophysiologist in 1970 (Spatial Summation and Temporal Summation of postsynaptic potentials, which may result in greater excitation or greater inhibition). Simultaneous inputs exist to assist and reach the threshold of neurons, generating an action potential (nerve impulses) to inhibit antagonistic muscle groups, and facilitate another muscle group.

2) Low Frequency EMS Currents: they achieve the inhibition of spasticity when applied directly to the motor point of the spastic agonist muscle.

3) Extracorporeal Shock Waves: they cause mechanical and biological effects, directly intervening on the intrinsic stiffness of the spastic muscle. The device simultaneously implements the postural procedures with sustained stretching, during the three-dimensional elongation procedure and in turn in shortening of the spastic muscle, using extracorporeal shock waves which provide greater viscoelasticity of the muscle tissues and the fasciae of the agonist and antagonist groups. This application achieves the reduction of spasticity, avoiding changes in the rheological properties during the evolution of the patient, for example the rigidity of the spastic muscle tissues, the shortening of the muscles, and muscular atrophy.

The intervention on the morphological component of spasticity using shock waves on the rigidity of tissues, acts on a factor of the vicious circle of spasticity, which is the constant contraction of the muscles. This, in addition to the exacerbation of involuntary contraction, causes greater rigidity as time passes, increased spasticity and changes in the rheological properties of muscles. Therefore, a longerlasting evolution with no elongation interventions, leads to shortening and loss of sarcomeres, as well as loss of collagen, reaching a restriction of the joint range and the consolidation of the joint itself or ossification. In the spastic muscles treated with shock waves, new collagen fibers are generated in the muscle tissue and elastin, so that when applying the radial shock waves the muscle is less susceptible to elongations, which causes the reduction or disappearance of spasms and clonus, allowing access to the physiological joint ranges.

4) Vibrators: these apply autogenous inhibition with intense and deep vibratory stimuli, between 30 to 1500 Hertz, which used on tendons and muscle-tendons, stimulating the Golgi tendon organ. Vibrators operate to provoke the GTO simultaneously during the stretching of the spastic muscles.

5) Rollers: they use deep mechanical pressure, encompassing the fasciae spastic muscle chains following the pattern of spasticity, simultaneously applied with sustained elongation. The elongations make the posture correction and also stimulate the Pacinian corpuscle, assisting in the proprioception information, reeducating towards the normal body schema and generating a cortical motor engram (stable neuronal interconnection).

6) Ultrasound: assists spastic and muscle-tendon musculature, provides plasticity and inhibition of the stretch myotatic reflex (hyperreflexia).

7) Thermotherapy: with laser or infrared it can be applied before or simultaneously to elongation having the advantages of improving circulation and facilitating the creation of collagen. With increasing body temperature, the elongation capacity is facilitated, enhancing the viscoelastic properties.

8) Magnetotherapy: repetitive magnetic stimuli have an antispastic influence. The effects achieved are less sensitivity in the spastic muscles, reduction of signs of hypertonia such as hyperreflexia or spontaneous muscle spasms, and it assists in slow and sustained passive elongations.

### Device conformation

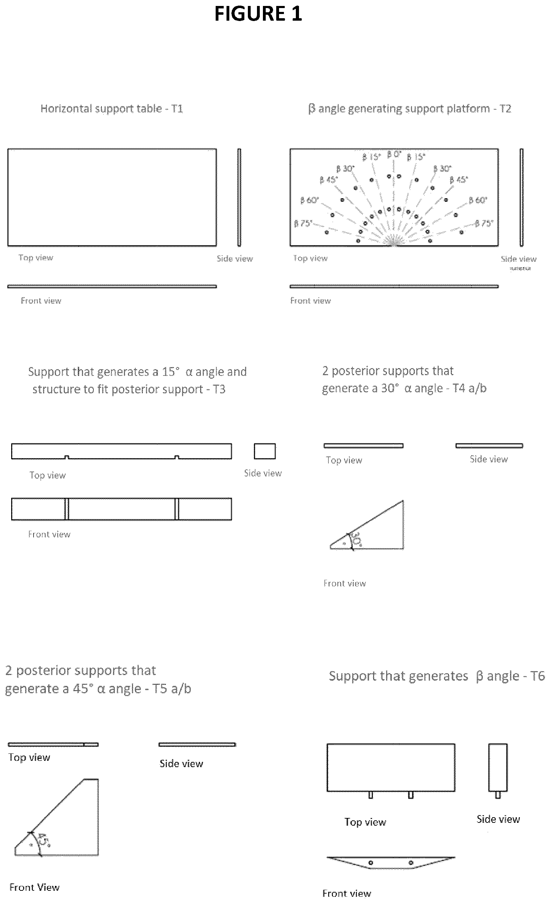

The first piece of the device of the present invention is the one that supports the entire structure, as shown in FIGURE 1. This part is placed on the floor and is called the horizontal support table (**T1**). To said horizontal support table **T1** a rectangular prism (**T3**) is attached, which is a support that fulfills two objectives, on the one hand, it generates the first alpha angle of 15° and it also has traces to fit the supports called triangular wedges, capable of generating alpha angles in the sagittal plane (**T4** and **T5**). The alpha angles are formed with the supporting platform (**T2**) on **T3**, **T4** and **T5** in the sagittal plane.

Triangular wedges **T4** and **T5** fit into **T3**, generating alpha angles of 30° (**T4**) and 45° (**T5**) respectively, as necessary, so that this primary system generates, in the sagittal plane, an α angle at 15° **T3** and is directly attached to **T1.**

On the platform **T2** where the patient places the foot to be treated, a secondary beta angle generator system is installed, which consists of a support (**T6**) which fits into **T2** and in turn fulfills the function of containment and location of the foot on the platform. Therefore, **T6** fits into the platform **T2** and generates the β angle, being able to increase or decrease it by 15° as necessary in the transverse plane. This combination of alpha α and beta β angles generates joint ranges when the patient's foot rests on the platform, achieving that the antispastic device of the present invention uses all three planes: sagittal in the tibiofibular-talar joint, frontal in the talocalcaneal or subtalar joint, and transverse in the midtalar joint.

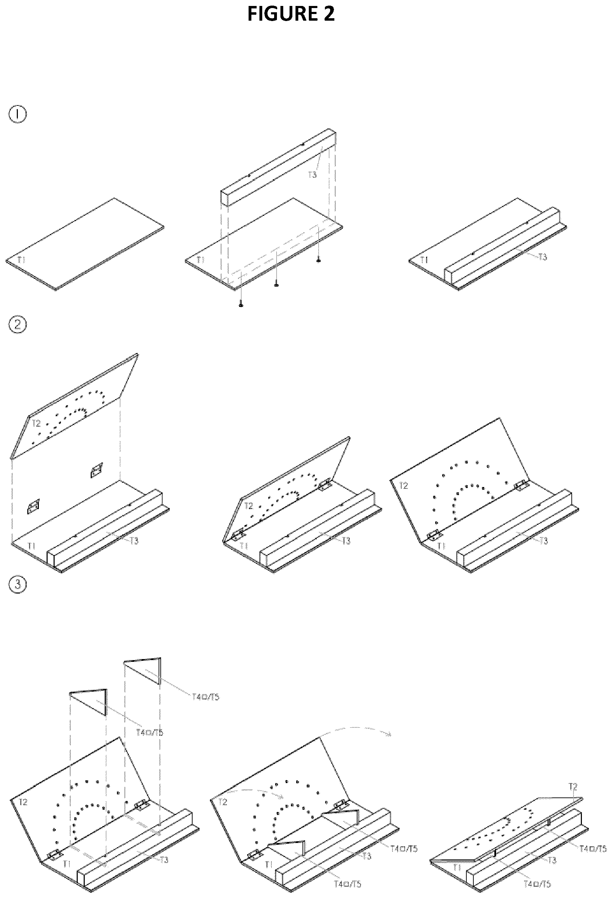

FIGURE 2 shows the assembly of the device to generate alpha angles:
1- On the horizontal support table **T1** the 15° alpha angle generator support **T3** is screwed.
2- Two hinges connecting the horizontal support table **T1** to the beta angle generator support platform **T2** are screwed.
3- The alpha angle generator supports are fitted, and may be of 30° (**T4**) or 45° (**T5**), and the UPPER (**T2**) are placed on them.

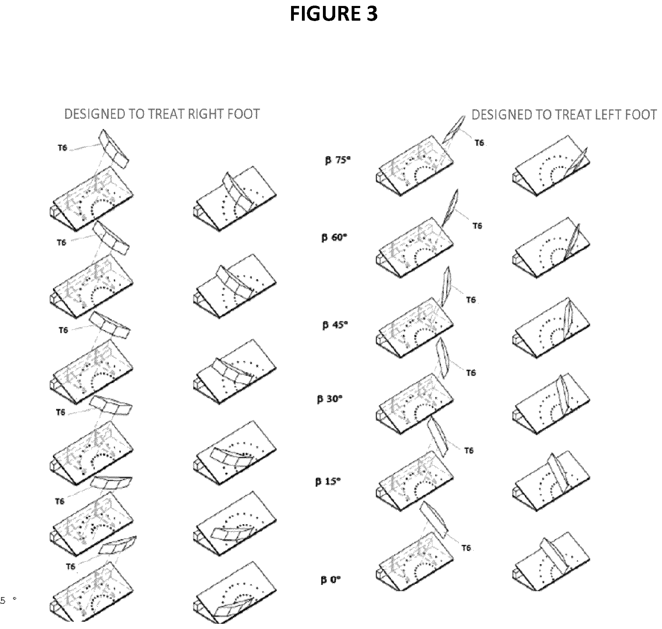

Likewise, FIGURE 3 shows the assembly of the device to generate beta angles in their configurations for the right foot and for the left foot, respectively.

The joint ranges (grid) in the device are defined according to the following:

| α | β | sin α cos β | Tibiofibular-talar joint | Talocalcaneal joint |
|---|---|---|---|---|
| 15° | 0° | 0.258 819 | 15° | 0° |
| | 15° | 0.250 000 | 14°.30' | 4° |
| | 30° | 0.224 144 | 13° | 7°30' |
| | 45° | 0.183 013 | 10°30' | 10°30' |
| | 60° | 0.129 410 | 7°30' | 13° |
| | 75° | 0.066 987 | 4° | 14°30' |
| | 90° | 0.000 000 | 0° | 15° |

| α | β | sin α cos β | Tibiofibular-talar joint | Talocalcaneal joint |
|---|---|---|---|---|
| 30° | 0° | 0.500 000 | 30° | 0° |
| | 15° | 0.482 963 | 27°30' | 7°30' |
| | 30° | 0.433 013 | 25° | 14°30' |
| | 45° | 0.353 554 | 20°30' | 20°30' |
| | 60° | 0.250 000 | 14°30' | 25° |
| | 75° | 0.129 410 | 7°30' | 27°30' |
| | 90° | 0.000 000 | 0° | 30° |

| α | β | sin α cos β | Tibiofibular-talar joint | Talocalcaneal joint |
|---|---|---|---|---|
| 45° | 0° | 0.707 107 | 45° | 0° |
| | 15° | 0.683 013 | 43° | 10°30' |
| | 30° | 0.612 372 | 38° | 20°30' |
| | 45° | 0.500 000 | 30° | 30° |
| | 60° | 0.353 554 | 20°30' | 38° |
| | 75° | 0.183 013 | 10°30' | 43° |
| | 90° | 0.000 000 | 0° | 45° |

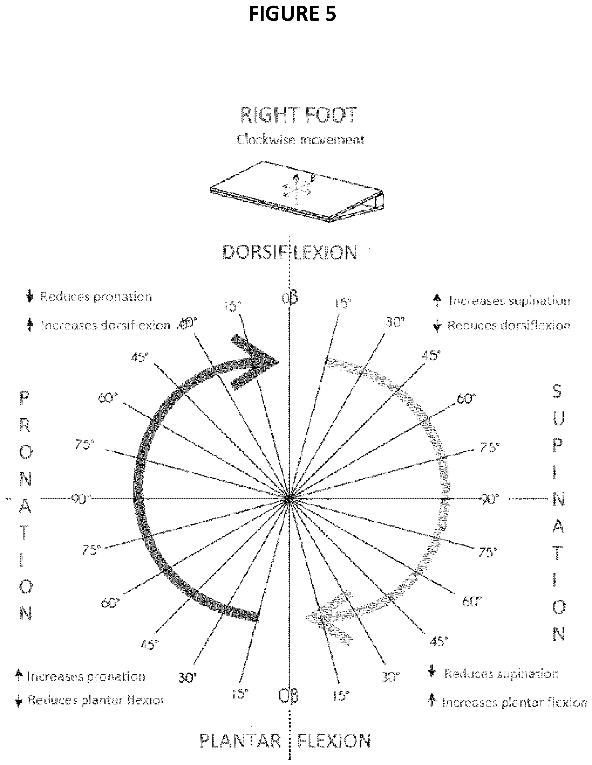

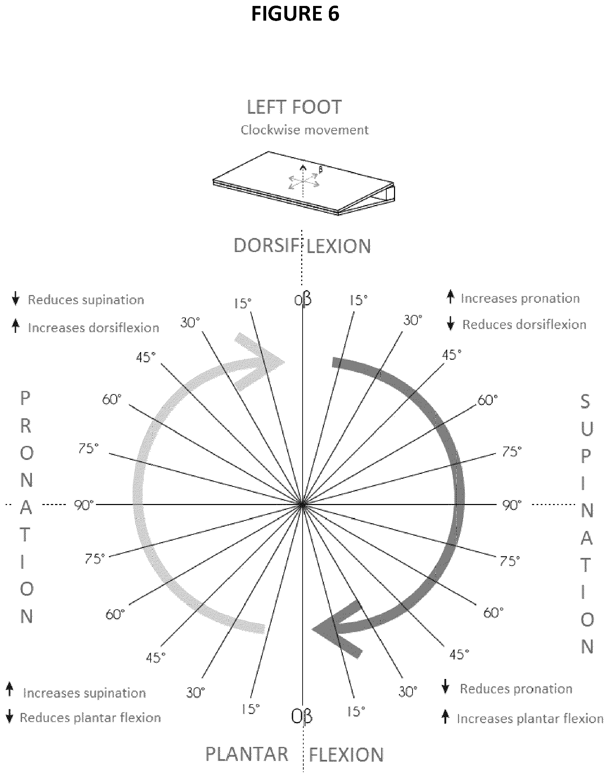

The grid shows how far the joint is moving, and the joint range diagrams shown in FIGURE 5 and FIGURE 6 show foot positions.

In turn, quadrants have different characteristics according to:

| | |
|---|---|
| RF ascending contralateral | RF ascending homolateral |
| LF ascending homolateral | LF ascending heterolateral |
| **LHD** | **RHD** |
| ascending | ascending |
| RF descending homolateral | RF descending contralateral |
| LF descending contralateral | LF descending homolateral |
| **RHD** | **LHD** |
| descending | descending |

To precisely use the ranges of the antispastic device, the patient undergoing an inhibition procedure must place the leg placed from the vertical in the frontal cut and the sagittal cut to treat the equinovarus foot. Thus, the alignment of the leg to be treated with the vertical plane is checked by the use of a vertical beam flashlight (VF): a straight, fine and vertical light is applied therefor. As references for the laser light, in the sagittal plane, it goes in the path of the greater trochanter behind the patella and in front of the external malleolus, and in the frontal plane, through the lower and upper center of the patella.

Further, the device includes an electronic mobility sensor in three dimensions to relocate the leg to be treated from the vertical.

The device knows the joint ranges analytically or systemically, obtaining the graduation of the elongation described in the grid, which is important for the inhibition, ranking and selection of one muscle over another, depending on the patient's clinical condition. Likewise, the antispastic device generates angles to be able to adapt to all clinical conditions of the spastic foot, using combinations of α 0° by β 0° until α 45° by β of 360°.

The angles described in the grid are repeated in each quadrant, therefore, changing quadrants results in the same ranges in each joint but in another position, depending on the variants:
- whether it is the right foot or the left foot,
- whether it is in the left or right quadrant,
- whether it is up or down,
- whether it is clockwise or counterclockwise.

For example, if the right foot is in the upper left quadrant with an alpha of 15° and beta of 30°, it is dorsiflexed at the tibiofibular-talar joint 13°, and pronation or valgus at the talocalcaneal joint 7°30'; and if it is in the upper right quadrant with an alpha of 15° and beta of 30°, it is dorsiflexed at the tibiofibular-talar joint 13°, and supination or varus at the talocalcaneal joint 7°30'.

In another example, if the left foot is in the left upper quadrant with an alpha of 15° and beta of 30°, it is dorsiflexed in the tibiofibular-talar joint 13°, of supination or varus in the talocalcaneal joint 7°30'; and if it is in the upper right quadrant an alpha of 15° and beta of 30°, it is dorsiflexed at the tibiofibular-talar joint 13°, and pronation or valgus at the talocalcaneal joint 7°30'.

### EXAMPLES OF EMBODIMENTS

The device of the present invention is designed to be used on both feet successively, so that the references to the left and right in each use of the device shall be those required by each patient. For example, a reference is the right half of the device (RHD), and the left half (LHD). Another reference of the patient shall be the location from an inclined plane in the sagittal plane, ascending the forefoot shall be higher than the hindfoot, and descending in the sagittal plane the forefoot shall be lower than the hindfoot.

The antispastic device of the present invention covers all necessary cases, therefore, when muscles are assessed and joint ranges are evaluated, they are loaded into the software and processed based on the data obtained.

Thus, by way of example, some particular arrangements of the device of the present invention are described in FIGURES 5 and FIGURE 6 covering the entire space for treating the spastic foot:
a- When the assessment is done and the patient achieves a neutral position or reduces beyond the neutral position in all planes, the device must be used heterolaterally ascending, so that, for example, the right foot (RF) uses the left half of the device (LHD) and the left foot (LF) uses the right half (RHD).
b- When the assessment is done and the patient reduces the equine and, in varus, does not reduce towards the neutral position in the frontal plane of the talocalcaneal joint, the rehabilitation procedure must be started with the LF using the beta ranges in the LHD in homolateral ascending position, and vice versa, the RF must start the procedure with RHD in homolateral ascending position. This will be part of the evolution process to reach rehabilitation, gaining joint ranges from varus to valgus or from supination to pronation. The goal is the inhibition of spasticity. In this example, the evolution shall lead to rising positions from the homolateral towards the use of the device in heterolateral in order to treat the equinovarus foot deformity. For example, the LF must achieve in the evolution of the rehabilitation treatment the use of the device with the beta angles clockwise and go from LHD to RHD and vice versa, the RF must use the device in an ascending position and advance the beta angle counterclockwise and go from the RHD to LHD. This scenario is the desirable in order to inhibit spasticity, eliminate Clonus, spasms and cover the greater joint ranges.
c- When the assessment of the equinovarus foot is made and the equinus is not reduced 90° of the sagittal plane of the tibiofibular-talar joint and if it reduces the varus in the frontal plane more than the neutral position in the subtalar or talocalcaneal joint, the use of the antispastic device with a central axis to the upper reference edge 0° of beta and with the foot in a descending position shall be necessary. When the equinovarus foot is not reduced, it is placed in the center of the upper edge (the highest and most central sector of the device), so that initially the patient shall have, in the flexed position, the forefoot lower than the hindfoot, and shall use the homolateral descending position, for example, with the RF descending and using the RHD, and the LF downwards and using the LHD, until reaching the plantigrade support at 90° in the tibiofibular-talar joint on the floor. During the rehabilitation process, it is desirable to continue towards dorsiflexion achieving greater inhibition of spasticity.
d- If the patient does not reduce the joint ranges and cannot be brought to the neutral position, for example, by not reducing the equine nor the varus, the procedure is initially performed in the same way on both feet, with the device in a downwards position and heterolaterally in a downwards position from top to bottom.

Possible designs of the device are described below, by way of example and not as a limitation:
1) A design is made with the alpha angles from 0° up to 90° in the sagittal plane, using the device in ascending position, with the beta angles from 0° to 90 ° in the transverse plane; 2 beta axes are generated at the lower edge of the platform, for example an axis on the left vertex of the platform, and in turn an axis on the right vertex of the platform, that is, 2 axes at the lower vertices with 90° of β angles, both axes spanning 180° of β angles. This way it can be used for each foot to be treated, allowing the patient's homolateral and heterolateral use.
2) A design is made with the alpha angles from 0° up to 90° in the sagittal plane, using the device in descent, with beta angles from 0° to 90° in the transverse plane; 2 beta axes are generated at the upper edge of the platform, for example an axis on the left vertex of the platform and an axis on the right vertex of the platform, that is, 2 axes at the upper vertices with 90° of β angles, both axes spanning 180° of β angles. This way it can be used for each foot to be treated, allowing the patient's homolateral and heterolateral use.
3) A design is made with the alpha angles from 0° to 90° in the sagittal plane. Thus, adding example 1 to 2, constitutes 4 beta axes at the vertices with 2 axes (left and right) on the upper edge in descent and 2 beta axes (left and right) on the lower edge in ascending position, so that each axis includes 90° of beta, which multiplied by 4 axes allows to cover all 360° of beta.
4) A design is made with the alpha angles from 0° up to 90° in the sagittal plane, and the beta angles on an axis on the left edge and also an axis on the right edge of the platform, the patient's support is approached from the lateral edges, that is to say lateral, being the reference to locate the patient in the examples 1) ascending and 2) in descent; from the upper half of the platform it is used in ascent and in turn the support will have from the lower half of the platform it is used in descent. The axes located at the sides generate 180° of beta angles on the platform at each edge, so that when the patient places his foot on the platform he will have on each axis 90° in ascent and 90° in descent.
5) A design is made with the alpha angles from 0° to 90° in the sagittal plane. The device meets the platform on the central axis, generating beta angles from 0° to 360 ° and alpha angles from 0° to 90°, which will treat both feet in a comprehensive manner. In this design, the device includes all the foot's postural needs, that is, the platform covers the entire space, assisting in any stage of the patient's evolution. The 90° by 360° combination of the device, as shown in FIGURE 5 (right foot clockwise) and as shown in FIGURE 6 (left foot clockwise) allows 16,200 different positions that result in 32,400 joint ranges (in the tibiofibular-talar and sub-talar joints) suitable to inhibit spasticity in the spastic foot.

## Claims

1. An antispastic device comprising:
- a horizontal support table (T1), which has the shape of a rectangular prism,
- a posterior supports insert structure (T3) which has the shape of a rectangular prism and is attached to the horizontal support table (T1) close to a posterior long edge of the horizontal support table (T1),
- two pairs of triangular wedges (T4,T5),
- a supporting platform (T2) which has the shape of a rectangular prism and which contains an embedded board (T6),
wherein
• one of the longer edges of the supporting platform (T2) and an anterior long edge of the horizontal support table (T1) which is opposite to the posterior long edge of the horizontal support table (T1) are connected to each other with two hinges,
• said posterior supports insert structure (T3) has traces into which the triangular wedges (T4,T5) can fit,
• different alpha angles can be generated in the sagittal plane by supporting the supporting platform (T2) on the posterior supports insert structure (T3) in or without combination with the triangular wedges (T4,T5),
• the embedded board (T6) generates in use beta angles in the transverse plane,
whereby the supporting platform (T2) can support a patient so that a three-dimensional rehabilitation procedure can be achieved covering the frontal, sagittal and transverse planes.

2. The antispastic device of claim 1, comprising a connection of the antispastic device to a PC tower and a 3D monitor.

3. The antispastic device of claims 1 or 2, **characterized in that** a baropodometric platform can be attached to it.

4. The antispastic device of any one of claims 1 to 3, wherein the antispastic device is configured to be used simultaneously with inputs selected from the group of: Russian currents, Australian currents, FES currents, TENS currents, excitomotor currents, diadynamic currents, low frequency EMS currents, extracorporeal shock waves, vibrators, rollers, ultrasound, thermotherapy, magnetotherapy and laser.

5. The antispastic device of any one of claims 1 to 4, wherein the antispastic device is configured to grade an elongation described in a grid such that the joint ranges are analytically or systemically known.

## Patentansprüche

1. Eine antispastische Vorrichtung, umfassend:
- einen horizontalen Stütztisch (T1), der die Form eines rechteckigen Prismas hat,
- eine posteriore Stützeinsatzstruktur (T3), die die Form eines rechteckigen Prismas hat und nahe zu einer posterioren langen Kante des horizontalen Stütztischs (T1) an dem horizontalen Stütztisch (T1) angebracht ist,
- zwei Paare dreiseitige Keile (T4, T5),
- eine Stützplattform (T2), die die Form eines rechteckigen Prismas hat und eine verankerte Platte (T6) enthält,
wobei
• eine der längeren Kanten der Stützplattform (T2) und eine anteriore lange Kante des horizontalen Stütztischs (T1), die der posterioren langen Kante des horizontalen Stütztischs (T1) gegenüberliegt, mittels zweier Gelenke miteinander verbunden sind,
• die posteriore Stützeinsatzstruktur (T3) Spuren aufweist, in die die dreiseitigen Keile (T4, T5) passen können,
• verschiedene Alpha-Winkel in der Sagittalebene durch Abstützen der Stützplattform (T2) auf der posterioren Stützeinsatzstruktur (T3) in Kombination mit oder ohne Kombination mit den dreiseitigen Keilen (T4, T5) erzeugt werden können,
• die verankerte Platte (T6) beim Gebrauch Beta-Winkel in der Transversalebene erzeugt,
wobei die Stützplattform (T2) einen Patienten stützen kann, sodass eine dreidimensionale Rehabilitationsbehandlung durchgeführt werden kann, die die Frontal-, Sagittal- und Transversalebenen abdeckt.

2. Die antispastische Vorrichtung nach Anspruch 1, umfassend eine Verbindung der antispastischen Vorrichtung mit einem PC-Tower und einem 3D-Monitor.

3. Die antispastische Vorrichtung nach Anspruch 1 oder 2, **dadurch gekennzeichnet, dass** eine baropodometrische Plattform daran befestigt werden kann.

4. Die antispastisches Vorrichtung nach einem der Ansprüche 1 bis 3, wobei sie derart ausgebildet ist, dass sie gleichzeitig mit Anregungen verwendet werden kann, die aus folgender Gruppe ausgewählt sind: Russische Ströme, australische Ströme, FES-Ströme, TENS-Ströme, exzitomotorische Ströme, diadynamische Ströme, niederfrequente EMS-Ströme, extrakorporale Stoßwellen, Vibratoren, Rollen, Ultraschall, Thermotherapie, Magnetfeldtherapie und Laser.

5. Die antispastische Vorrichtung nach einem der Ansprüche 1 bis 4, wobei sie derart ausgebildet ist, dass sie das Einstufen einer in einem Raster beschriebenen Dehnung ermöglicht, sodass die Gelenkbereiche analytisch oder systematisch bekannt sind.

## Revendications

1. Dispositif antispastique comprenant :
- une table de support horizontale (T1), qui a la forme d'un prisme rectangulaire,
- une structure d'insertion de supports postérieurs (T3) qui a la forme d'un prisme rectangulaire et qui est fixée à la table de support horizontale (T1) à proximité d'un bord long postérieur de la table de support horizontale (T1),
- deux paires de cales triangulaires (T4,T5),
- une plate-forme de support (T2) qui a la forme d'un prisme rectangulaire et qui contient une planche encastrée (T6),
dispositif dans lequel
- l'un des bords longs de la plate-forme de support (T2) et un bord long antérieur de la table de support horizontale (T1) qui est opposé au bord long postérieur de la table de support horizontale (T1) sont reliés l'un à l'autre par deux charnières,
- ladite structure d'insertion des supports postérieurs (T3) présente des traces dans lesquelles les cales triangulaires (T4,T5) peuvent s'insérer,
- différents angles alpha peuvent être générés dans le plan sagittal en soutenant la plate-forme de support (T2) sur la structure d'insertion de supports postérieurs (T3) en combinaison ou non avec les cales triangulaires (T4, T5),
- la planche encastrée (T6) génère en cours d'utilisation des angles bêta dans le plan transversal,
la plate-forme de support (T2) pouvant soutenir un patient de sorte qu'une procédure de rééducation tridimensionnelle couvrant les plans frontal, sagittal et transversal peut être réalisée.

2. Dispositif antispastique selon la revendication 1, comprenant une connexion du dispositif antispastique à une tour PC et à un écran 3D.

3. Dispositif antispastique selon la revendication 1 ou 2, **caractérisé en ce qu'**une plate-forme baropodométrique peut y être fixée.

4. Dispositif antispastique selon l'une quelconque des revendications 1 à 3, dans lequel le dispositif antispastique est configuré pour être utilisé simultanément avec des entrées choisies dans le groupe des : courants russes, courants australiens, courants FES, courants TENS, courants excitomoteurs, courants diadynamiques, courants EMS basse fréquence, ondes de choc extracorporelles, vibrateurs, rouleaux, ultrasons, thermothérapie, magnétothérapie et laser.

5. Dispositif antispastique selon l'une quelconque des revendications 1 à 4, dans lequel le dispositif antispastique est configuré pour classer une élongation décrite dans une grille de sorte que les amplitudes articulaires soient connues de manière analytique ou systémique.
